## Europäisches Patentamt
## European Patent Office
## Office européen des brevets

(11) Publication number: **0 086 548**
**B1**

(12) **EUROPEAN PATENT SPECIFICATION**

(45) Date of publication of patent specification: **20.05.87**

(51) Int. Cl.⁴: **C 12 N 15/00**

(21) Application number: **83200301.6**

(22) Date of filing: **23.03.81**

(60) Publication number of the earlier application in accordance with Art. 76 EPC: **0 036 776**

(54) **A method of cleaving double stranded DNA.**

(30) Priority: **24.03.80 US 133296**

(43) Date of publication of application:
**24.08.83 Bulletin 83/34**

(45) Publication of the grant of the patent:
**20.05.87 Bulletin 87/21**

(84) Designated Contracting States:
**AT BE CH DE FR GB IT LI LU NL SE**

(56) References cited:
**EP-A-0 013 830**
**EP-A-0 043 980**
**EP-A-0 048 970**

**NUCLEIC ACIDS RESEARCH, vol. 8, no. 18, 1980, pages 4057-4074, IRL Press Ltd., London, GB. D.V. GOEDDEL et al.: "Synthesis of human fibroblast interferon by E. coli"**

(73) Proprietor: **GENENTECH, INC.**
**460 Point San Bruno Boulevard**
**South San Francisco California 94080 (US)**

(72) Inventor: **Kleid, Dennis G.**
**724 Costa Rica Avenue**
**San Mateo California 94404 (US)**
Inventor: **Yansura, Daniel G.**
**125 Pioche**
**San Francisco California 94134 (US)**
Inventor: **Heyneker, Herbert L.**
**2621 Easton Drive**
**Burlingame California 94010 (US)**
Inventor: **Miozzari, Giuseppe F.**
**Im Feverbusch 9**
**CH-4310 Augarten Rheinfelder (CH)**

(74) Representative: **Armitage, Ian Michael et al**
**MEWBURN ELLIS & CO. 2/3 Cursitor Street**
**London EC4A 1BQ (GB)**

Courier Press, Leamington Spa, England.

**Description**

This invention relates to recombinant DNA technology, and, together with European Patent application 85100548.8 (EP—A—154133) is divided out of our co-pending European Patent Application No. 81301227.5 (EP—A—36776) (see also GB—A—2073203).

Recombinant DNA procedures often involve cleaving double stranded DNA in a controlled manner, but restriction enzymes only cleave the DNA at recognition sites (i.e. nucleotide sequences) specific to the enzyme in question. The present invention provides means for cleaving double-stranded DNA at any desired point, even absent a restriction enzyme site, a technique useful in, among other things, the creation of trp operons having attenuator deletions other than those previously obtained by selection of mutants. The invention also includes recombinant DNA vectors produced by using this technique, host cells containing such vectors, and heterologous polypeptides produced by expression from such cells.

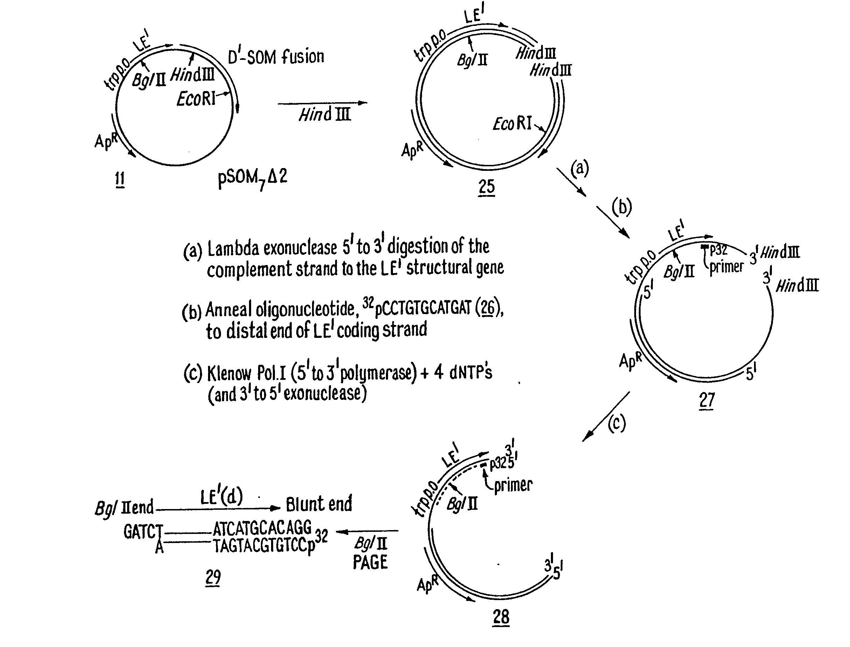

The present invention will be more particularly illustrated in the following detailed description, with reference to the accompanying drawing which illustrates the procedure of the present invention.

In general terms, double-stranded DNA is converted to single-stranded DNA in the region surrounding the intended cleavage point, as by reaction with lambda exonuclease. A synthetic or other single-stranded DNA primer is then hybridized to the single-stranded length earlier formed, by Watson-Crick base-pairing, the primer sequence being such as to ensure that the 5' end thereof will be coterminous with the nucleotide on the first strand just prior to the intended cleavage point. The primer is next extended in the 3' direction by reaction with DNA polymerase, recreating that portion of the original double-stranded DNA prior to the intended cleavage that was lost in the first step. Simultaneously or thereafter, the portion of the first strand beyond the intended cleavage point is digested away. To summarize, where "v" marks the intended cleavage point:

|   |   |   |
|---|---|---|
| a) | ⌄ | intended cleavage point "v" |
| b) | ⌄ | made single stranded around "v" |
| c) | ⌄ | primer hybridization |
| d) | ⌄ | extension from primer |
| e) | ⌄ | single strand digestion |

In the most preferred embodiment, steps (d) and (e) are performed simultaneously, using a polymerase that simultaneously digests the protruding single stranded end in the 3'→5' direction and extends the primer (in the presence of dATP, dGTP, dTTP and dCTP) in the 5'→3' direction. The material preferred for this purpose is Klenow Polymerase I, i.e., that fragment obtained by proteolytic cleavage of DNA Polymerase I which contains the 5'→3' polymerizing activity and the 3'→5' exonucleolytic activity of the parental enzyme, yet lacks its 5'→3' exonucleolytic activity (A. Kornberg, *DNA Synthesis*, 98, W. H. Freeman and Co., SFO (1974)).

Using the procedure just described, attenuator deletions may be made in any desired manner in a trp operon-containing plasmid first linearized by, e.g., cleavage at a restriction site downstream from the point at which the molecule is to be blunt-ended ("v" above). Recircularization following deletion of the attenuator region may be effected, e.g., by blunt end ligation or other manners which will be apparent to the art-skilled. The invention is used in procedures described in parent specification EP—A—0036776 and in European Patent application 85100548.8 (EP—A—154133) (see also GB—A—2073203).

With reference to the drawing plasmid pSom7 Δ2 (see EP—A—36776 and European Patent application 85100548.8 (EP—A—154133) see also GB—A—2073203) was Hind III digested followed by digestion with lambda exonuclease (a 5' to 3' exonuclease) under conditions chosen so as to digest beyond the Bgl II restriction site within the LE' encoding region. 20 µg of Hind III-digested pSom 7 Δ2 was dissolved in buffer [20 mM glycine buffer, pH 9.6, 1 mM MgCl₂, 1 mM β-mercaptoethanol]. The resulting mixture was treated with 5 units of lambda exonuclease for 60 minutes at room temperature. The reaction mixture obtained was then phenol extracted, chloroform extracted and ethanol precipitated.

In order ultimately to create an EcoRI residue at the distal end of the LE' gene fragment a primer

[32]pCCTGTGCATGAT was synthesized by the improved phosphotriester method (R. Crea *et al.*, Proc Nat'l Acad Sci USA *75*, 5765 [1978]) and hybridized to the single stranded end of the LE' gene fragment resulting from lambda exonuclease digestion. The hybridization was performed as next described.

20 µg of the lambda exonuclease-treated Hind III digestion product of plasmid pSom7 Δ2 was dissolved in 20 µl H₂O and combined with 6 µl of a solution containing approximately 80 picomoles of the 5'-phosphorylated oligonucleotide described above. The synthetic fragment was hybridized to the 3' end of the LE' coding sequence and the remaining single strand portion of the LE' fragment was filled by the Klenow polymerase I procedure described above, using dATP, dTTP, dGTP and dCTP.

The reaction mixture was heated to 50°C and let cool slowly to 10°C, whereafter 4 µl of Klenow enzyme were added. After 15 minute room temperature incubation, followed by 30 minutes incubation at 37°C, the reaction was stopped by the addition of 5 µl of 0.25 molar EDTA. The reaction mixture was phenol extracted, chloroform extracted and ethanol precipitated. The DNA was subsequently cleaved with the restriction enzyme Bgl II. The fragments were separated by PAGE. An autoradiogram obtained from the gel revealed a $^{32}$P-labelled fragment of the expected length of approximately 470 bp, which was recovered by electroelution. As outlined, this fragment LE'(d) has a Bgl II and a blunt end coinciding with the beginning of the primer.

This can then be used e.g. as described in EP—A—36776, EP—A—154133 (European Patent Application 85100548.8) (see also GB—A—2073203) to create an expression vector containing the LE'(d) fragment (29) under the control of a promoter (trp promoter-operator), in which vector can be functionally inserted the coding sequence of a heterologous polypeptide such as thymosin alpha one, human proinsulin, or the A or B chain of human insulin. The resulting expression vector can then be transformed into a suitable host (*E. coli* strain 294) to express the heterologous polypeptide.

## Claims

1. A method of cleaving double stranded DNA at any given point which comprises:

(a) converting the double stranded DNA to single-stranded DNA in a region surrounding the intended cleavage site;

(b) hybridizing to the single-stranded region formed in step (a) a complementary primer length of single-stranded DNA, the 5' end of the primer lying opposite the nucleotide adjoining the intended cleavage site;

(c) restoring that portion of the second strand eliminated in step (a) which lies in the 3' direction from said primer by reaction with DNA polymerase in the presence of adenine, thymine, guanine and cytosine-containing deoxynucleotide triphosphates; and

(d) digesting the remaining single-stranded length of DNA which protrudes beyond the intended cleavage point.

2. The method of claim 1 wherein steps (c) and (d) are performed simultaneously by reaction with DNA polymerase which polymerizes in the direction of 5'→3', is exonucleolytic in the direction of 3'→5', but non-exonucleolytic in the direction of 5'→3'.

3. The method of claim 2 wherein the polymerase is Klenow Polymerase I.

4. The use of a method of any of claims 1, 2 and 3 in the creation of DNA expression vectors.

5. The use of the method of claim 4 in the production of host cells transformed with said expression vectors.

6. The use of the method of claim 5 in the production of heterologous polypeptides by said transformed cells.

7. The use of a method of any of claims 1, 2 and 3 in the creation of recombinant DNA vectors.

8. The use of a method of any one of claims 1, 2 and 3 in the production of polypeptides by recombinant DNA technology.

## Patentansprüche

1. Verfahren zum Spalten von Doppelstrang-DNS an jedem beliebigen Punkt, umfassend

(a) Umwandeln der Doppelstrand-DNS zu Einzelstrang-DNS in einem Bereich, der die beabsichtigte Spaltungsstelle umgibt;

(b) Hybridisieren einer komplementären Primerlänge von Einzelstrang-DNS an den in Schritt (a) gebildeten Einzelstrangbereich, wobei das 5'-Ende des Primers gegenüber dem Nukleotid liegt, das an die beabsichtigte Spaltungsstelle angrenzt;

(c) Wiederherstellung jenes Abschnittes des in Schritt (a) eliminierten zweiten Stranges, der in 3'-Richtung von dem genannten Primer liegt, durch Reaktion mit DNS-Polymerase in Gegenwart von Adenin, Thymin, Guanin und Cytosin enthaltenden Deoxynukleotidtriphosphaten; und

(d) Digerieren der verbleibenden Einzelstranglänge von DNS, die über den beabsichtigten Spaltungspunkt hinausragt.

2. Das Verfahren nach Anspruch 1, worin die Schritte (c) und (d) gleichzeitig durch Reaktion mit DNS-Polymerase durchgeführt werden, die in Richtung 5'→3' polymerisiert und exonukleolytisch in Richtung 3'→5', jedoch nichtexonukleolytisch in Richtung 5'→3' ist.

3. Das Verfahren nach Anspruch 2, worin die Polymerase Klenow-Polymerase I ist.

4. Die Anwendung eines Verfahrens nach einem der Ansprüche 1, 2 und 3 zur Schaffung von DNS-Expressionsvektoren.

5. Die Anwendung des Verfahrens nach Anspruch 4 zur Herstellung von Wirtszellen, die mit den genannten Expressionsvektoren transformiert sind.

6. Die Anwendung des Verfahrens nach Anspruch 5 zur Herstellung von heterologen Polypeptiden mittels der genannten transformierten Zellen.

7. Die Anwendung eines Verfahrens nach einem der Ansprüche 1, 2 und 3 zur Schaffung von rekombinanten DNS-Vektoren.

8. Die Anwendung eines Verfahrens nach einem der Ansprüche 1, 2 und 3 bei der Herstellung von Polypeptiden mittels rekombinanter DNS-Technologie.

**Revendications**

1. Procédé de clivage d'ADN bicaténaire en tout point donné qui comprend:

(a) la conversion de l'ADN bicaténaire en ADN monocaténaire dans une région entourant le site souhaité de clivage;

(b) l'hybridation à la région monocaténaire formée à l'étape (a) d'une longueur d'amorce complémentaire d'ADN monocaténaire, l'extrémité 5' de l'amorce se trouvant face au nucléotide adjacent au site souhaité de clivage;

(c) la restauration de la partie du second brin éliminée à l'étape (a) qui se trouve en direction 3' à partir de ladite amorce par réaction avec l'ADN polymérase en présence de désoxynucléotide triphosphates contenant de l'adénine, de la thymine, de la guanine et de la cytosine; et

(d) la digestion de la longueur monocaténaire restante de l'ADN qui dépasse au delà du brin souhaité de clivage.

2. Procédé de la revendication 1 où les étapes (c) et (d) sont accomplis simultanément par réaction avec l'ADN polymérase qui polymérise en direction de 5'→3', est exonucléolytique dans la direction de 3'→5' mais non exonucléolytique dans la direction de 5'→3'.

3. Procédé de la revendication 2 où la polymérase est la polymérase Klenow I.

4. Utilisation du procédé selon l'une quelconque des revendications 1, 2 et 3 dans la création de vecteurs d'expression d'ADN.

5. Utilization du procédé de la revendication 4 pour la production de cellules hôtes transformées par lesdits vecteurs d'expression.

6. Utilisation du procédé de la revendication 5 pour la production de polypeptides hétérologues par lesdites cellules transformées.

7. Utilisation d'un procédé selon l'une quelconque des revendications 1, 2 et 3 dans la création de vecteurs d'ADN recombinant.

8. Utilisation d'un procédé selon l'une quelconque des revendications 1, 2 et 3 dans la production de polypeptides par technologie d'ADN recombinant.

D'-SOM fusion

pSOM₇Δ2

11

*Hind* III →

25

(a)

(b)

(a) Lambda exonuclease 5' to 3' digestion of the complement strand to the LE' structural gene

(b) Anneal oligonucleotide, ³²pCCTGTGCATGAT (26), to distal end of LE' coding strand

(c) Klenow Pol.I (5' to 3' polymerase) + 4 dNTP's (and 3' to 5' exonuclease)

27

(c)

*Bgl* II end ———— LE'(d) ———→ Blunt end

GATCT————ATCATGCACAGG ₃₂
    A ————TAGTACGTGTCCp³²

29

*Bgl* II
PAGE

28

0 086 548